# EUROPEAN PATENT SPECIFICATION

(11) **EP 4 068 993 B1**
(45) Date of publication and mention of the grant of the patent: **14.02.2024**
(21) Application number: 20816247.9
(22) Date of filing: 04.12.2020
(51) Int. Cl.: A23L 33/115, A23L 33/00, A61K 31/215, A61P 3/04

(54) **INFANT FORMULA WITH SPECIAL LIPID ARCHITECTURE FOR IMPROVING POSTNATAL GROWTH OF INFANTS BORN TO OVERWEIGHT AND OBESE MOTHERS**
SÄUGLINGSANFANGSNAHRUNG MIT SPEZIELLER LIPIDARCHITEKTUR ZUR VERBESSERUNG DES POSTNATALEN WACHSTUMS VON SÄUGLINGEN, DIE VON ÜBERGEWICHTIGEN UND ADIPÖSEN MÜTTERN GEBOREN WURDEN
FORMULE POUR NOURRISSONS DOTÉE D'UNE ARCHITECTURE DE LIPIDES SPÉCIALE POUR AMÉLIORER LA CROISSANCE POST-NATALE DE NOURRISSONS NÉS DE MÈRES EN SURPOIDS ET OBÈSES

(30) Priority: 05.12.2019 EP 19213823
(43) Date of publication of application: 12.10.2022
(73) Proprietor: N.V. Nutricia, 2712 HM Zoetermeer (NL)
(72) Inventor: SCHOEN, Stefanie, 3584 CT Utrecht (NL); ACTON, Dennis Stanley, 3584 CT Utrecht (NL)
(74) Representative: Nederlandsch Octrooibureau
(86) International application number: PCT/EP2020/084632
(87) International publication number: WO 2021/110916

(56) References cited:
- WO-A1-2012/173467
- WO-A1-2017/064304
- US-A1- 2010 111 915
- MATTHEW W. GILLMAN ET AL: "Developmental Origins of Childhood Overweight: Potential Public Health Impact", OBESITY RESEARCH, vol. 16, no. 7, 1 July 2008 (2008-07-01), pages 1651-1656, XP055705504, US ISSN: 1930-7381, DOI: 10.1038/oby.2008.260
- R. C. WHITAKER: "Predicting Preschooler Obesity at Birth: The Role of Maternal Obesity in Early Pregnancy", PEDIATRICS, vol. 114, no. 1, 1 July 2004 (2004-07-01), pages e29-e36, XP055705508, US ISSN: 0031-4005, DOI: 10.1542/peds.114.1.e29
- CATHARINE R. GALE ET AL: "Maternal Size in Pregnancy and Body Composition in Children", JOURNAL OF CLINICAL ENDOCRINOLOGY AND METABOLISM, vol. 92, no. 10, 1 October 2007 (2007-10-01), pages 3904-3911, XP055705513, US ISSN: 0021-972X, DOI: 10.1210/jc.2007-0088
- PATRICK M CATALANO ET AL: "Perinatal risk factors for childhood obesity and metabolic dysregulation", THE AMERICAN JOURNAL OF CLINICAL NUTRITION, vol. 90, no. 5, 16 September 2009 (2009-09-16), pages 1303-1313, XP055705515, US ISSN: 0002-9165, DOI: 10.3945/ajcn.2008.27416

## Description

### FIELD OF THE INVENTION

The invention relates to nutrition for infants, in particular infant formula, intended to be used as a source of nutrition.

### BACKGROUND OF THE INVENTION

Human milk is the uncontested gold standard concerning infant nutrition. However, in some cases breastfeeding is inadequate or unsuccessful for medical reasons or because of a choice not to breastfeed. For such situations infant or follow on formulas have been developed. Commercial infant formulas are commonly used today to provide supplemental or sole source of nutrition early in life. These formulas comprise a range of nutrients to meet the nutritional needs of the growing infant, and typically include fat, carbohydrate, protein, vitamins, minerals, and other nutrients helpful for optimal infant growth and development. Commercial infant formulas are designed to mimic, as closely as possible, the composition and function of human milk.

Since long it has been appreciated that breastfed infants have a different weight gain pattern ortrajectory compared to formula-fed infants. After the first week of life, in which breastfed infants initially tend to lose more weight than formula-fed infants and take slightly longer to regain their birth weight, the weight gain patterns are similar between breastfed and formula-fed infants for the first 4 months of life. Breastfed infants tend to have slightly higher weight at 3 months age (Andres et al, 2013, J Pediatrics 163: 49-54). After about 4 months of age, the rate of weight gain diverges markedly between breastfed and formula-fed infants. The difference in average weight at 12 months approximates up to 500-650 g (Dewey et al., 1993, Am J Clin Nutr 57: 140-145; Dewey et al, 1992 Pediatrics 89:1035). Li et al, 2010, Pediatrics 125:e1386-e1393 discloses that infants that are bottle-fed in early infancy are more likely to empty the bottle or cup in late infancy than those who are fed directly at the breast. Bottle-feeding, regardless of the type of milk, is distinct from feeding at the breast in its effect on infants' self-regulation of milk intake. Breast fed infants have a decreased chance of becoming obese later in life, compared to standard formula fed infants.

Obesity is a major health problem in the Western world. It is a medical condition in which excess fat has accumulated to the extent that it may have an adverse effect on health, leading to reduced life expectancy and it is associated with many diseases, particularly heart disease and type 2 diabetes. A higher pre-pregnancy body mass index (BMI) is associated with an increased risk of gestational diabetes mellitus which in turn is associated with a higher risk for perinatal morbidity as well as childhood obesity, retarded psychomotor development and early onset of type 2 diabetes mellitus in the offspring (Torloni M et al. 2009, Obesity reviews 10: 194 - 203).

Children that are born to overweight and obese women are themselves more often overweight and obese and have an increased risk for cardiometabolic diseases themselves. Compared to children of mothers with a normal weight before pregnancy, children of overweight mothers grow faster in weight and BMI during the first year of life (Oostvogels A et al. 2017, Early human development 113: 62 -70). Also later in life this effect is apparent: a higher pre-pregnancy BMI is associated with a higher BMI in the offspring of these mothers in adolescence and an adverse adolescent cardio-metabolic profile (Gaillard R et al. 2016, BJOG 123: 207 - 216). Infants born to an overweight or obese mother are thus at risk of an unbalanced postnatal growth trajectory.

Human milk lipids are known have a distinct physical structure composed of large lipid globules with an average mode diameter of about 4 µm existing of a triglyceride core coated by a tri-layer of membranes, the milk fat globule membrane (MFGM). The diameter of lipid droplets in standard infant formula is typically about 0.3-0.5 µm due to the industrial processing procedures to achieve stable and reproducible end products, and the lipid droplets are not surrounded by MFGM but mostly by proteins such as casein. Infant formula with lipid globules with an architecture more similar to the lipid globules in human milk have been described.

WO 2017/064304 describes the comparison of the growth trajectory of infants receiving infant formulae with large lipid globules coated with phospholipids with the growth trajectory of infants from the breastfed reference group when looking at weight and body mass index (BMI) at 12 months of age.. WO 2015/065193 discloses nutritional compositions comprising specifically designed lipid globules for preterm infants, small for gestational age infants and infants with retarded growth due to physical or mental stress after birth, for promoting catch up growth and/or improved body composition. In WO 2012/173467 the use of specifically designed lipid component with optimal fatty acid profile, an enhanced portion of the palmitic acid residues at the sn-2 position and present as lipid globules with a certain size and/or coating is disclosed for an early in life diet for improving the development of a healthy body composition, in particular prevention of obesity, later in life. WO 2010/0027258 and WO 2010/0027259 relate to infant formulas with large lipid globules coated with phospholipids for the prevention of obesity later in life.

The present invention aims to provide infant nutrition with a lipid component beneficially affecting the postnatal growth trajectory or BMI development in an infant that is born to an overweight or obese mother.

### SUMMARY OF THE INVENTION

The inventors compared the BMI during the first 12 months of life of 3 groups of healthy term infants born to a mother being classified based on her pre-pregnancy BMI as either underweight, normal weight, overweight or obese. One group was a non-randomised reference group of breastfed infants, one group received an experimental infant formula comprising a lipid component in the form of large lipid globules coated with phospholipids, and one group received a control infant formula without a lipid component in the form of large lipid globules coated with phospholipids, but in the form of standard lipid globules with a mode diameter based on volume of about 0.3-0.5 µm coated with protein. Both formulas were administered up to 17 weeks of life. The control and experimental milk formulas were similar in caloric content, as well as in lipid, carbohydrate and protein content.

The inventors found that when analysing the BMI for the whole study period up to 12 months of age in both the intention to treat (ITT) and in the per protocol (PP) population that the BMI of the group of infants that received the experimental formula was significantly lower than the BMI of the group of infants that received the control infant formula for both the groups of infants that were born to mothers with a normal pre-pregnancy weight and for infants that were born to mothers that were overweight or obese before pregnancy. The BMI of the infants that were fed the experimental formula were more comparable to the BMI of breastfed infants, an effect that was particularly strong in infants that were born to mothers that were overweight or obese before pregnancy. In the latter group of infants, the BMI trajectory became comparable to the BMI trajectory of breastfed infants that were born to mothers with a normal pre-pregnancy body weight. The group of infants receiving the control formula on the other hand showed a higher BMI at 4 and at 12 months, when compared to the breastfed group. Furthermore, the group receiving control formula showed a higher BMI, when compared with the group fed with the experimental formula at 12 months.

The inventors surprisingly found that between baseline and 4 months of age the infants born from an overweight or obese mother fed the experimental formula had a significantly lower BMI gain than the infants born from an overweight or obese mother that were fed the control formula. The effect of the experimental diet on BMI gain was found to be stronger and earlier apparent, in particular at 4 months, in the infants born from overweight and obese mothers than in infants born to mothers having a normal pre-pregnancy weight.

Overall this indicates that feeding an infant formula with a similar caloric content and macro-ingredient content but having a different architecture of lipid globules can have a different and beneficial effect on the growth pattern or growth trajectory early in life, rendering it advantageously more similar to the growth pattern or trajectory early in life of breastfed infants born to mothers that have a normal weight before pregnancy. Infants receiving the experimental formula showed a BMI pattern and a postnatal growth trajectory that is more comparable to infants that are breastfed as compared to infants that received the control formula, the effect being surprisingly more prominent in the infants born to a mother that was overweight or obese pre-pregnancy. This is postulated to be due to the difference in the way the lipid component of the composition is configured, namely in a way more resembling human milk lipid globules, by being larger in size and coated with phospholipids.

### DETAILED DESCRIPTION OF THE INVENTION

The present invention concerns a method for improving the postnatal growth trajectory or body development in an infant that is born to a mother that is overweight or obese at the time of conception (pre-pregnancy) of the infant, said method comprising feeding said infant a nutritional composition selected from an infant formula and a follow-on formula comprising carbohydrates, protein and lipid,
i) wherein the lipid comprises linoleic acid and alpha-linolenic acid in a weight ratio of 2 to 20,
ii) the lipid comprises at least 10 wt.% palmitic acid based on total lipid, and at least 15 wt.% of the palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

In other words, the present invention concerns a nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids,
for use in improving the postnatal growth trajectory or body development in an infant that is born to a mother that is overweight or obese at the time of conception of the infant.

The invention can also be worded as the use of lipid globules in the manufacture of a nutritional composition selected from an infant formula and a follow on formula for use in improving the postnatal growth trajectory or body development in an infant that is born to a mother that is overweight or obese at the time of conception of the infant, wherein the nutritional composition comprises carbohydrates, protein and lipid, wherein,
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

It may be considered that improvement of the postnatal growth trajectory or body development in an infant that is born to a mother that is overweight or obese at the time of conception of the infant encompasses a non-therapeutic effect. In view thereof, the present invention can also be considered as relating to a method for non-therapeutic, or a non-therapeutic method for, improvement of the postnatal growth trajectory or body development in an infant that is born to a mother that is overweight or obese at the time of conception of the infant by administration of a nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

The present invention concerns a method for preventing or reducing the risk of an unbalanced postnatal growth trajectory or body development in an infant at risk of having an unbalanced postnatal growth trajectory or body development, said infant being born to a mother that is overweight or obese at the time of conception of the infant, said method comprising feeding said infant a nutritional composition selected from an infant formula and a follow on formula comprising carbohydrates, protein and lipid,
i) wherein the lipid comprises linoleic acid and alpha-linolenic acid in a weight ratio of 2 to 20,
ii) the lipid comprises at least 10 wt.% palmitic acid based on total lipid, and at least 15 wt.% of the palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

The invention also concerns a nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids,
for use in preventing or reducing the risk of an unbalanced postnatal growth trajectory or body development in an infant at risk of having an unbalanced postnatal growth trajectory or body development, said infant being born to a mother that is overweight or obese at the time of conception of the infant.

The invention can also be worded as the use of lipid globules in the manufacture of a nutritional composition selected from an infant formula and a follow on formula for use in preventing or reducing the risk of an unbalanced postnatal growth trajectory or body development in an infant at risk of having an unbalanced postnatal growth trajectory or body development, said infant being born to a mother that is overweight or obese at the time of conception of the infant, wherein the nutritional composition comprises carbohydrates, protein and lipid, wherein,
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

In a preferred embodiment of the method, use or composition for use according to the invention, the growth trajectory or body development is the growth trajectory or body development of the first 12 months of life of the infant.

In the context of the present invention, the infant formula or follow on formula is not native cow's milk or human milk.

In the context of the present invention a mother that is overweight or obese at the time of conception of the infant is based on the BMI of the mother before pregnancy. In one embodiment, the BMI before pregnancy is defined as the BMI as measured in the time period at least six months before conception.

In this specification, the following terms have the meanings assigned to them here below (Chiu M et al. Diabetes Care, 2011, 34:1741-1748):
BMI is defined as the body mass divided by the square of the body height, and is expressed in units of kg/m². The BMI broadly categorizes a person as underweight, normal weight, overweight, or obese according to the calculated BMI value.

Underweight is defined as a BMI < 18.5 kg/m² for all women.

A normal weight for Asian women is considered a BMI ≥185 and <23 kg/m², for non-Asian women, a normal weight is a BMI ≥18.5 and <25 kg/m².

Overweight for Asian women is considered a BMI ≥ 23 and < 27.5 kg/m², for non-Asian women, overweight is a BMI ≥ 25 and <30 kg/m².

Obese for Asian women is considered a BMI ≥ 27.5 kg/m², for non-Asian women, obese is a BMI ≥ 30 kg/m².

### Lipid globules

According to the present invention, the nutritional composition comprises lipid globules. When in liquid form these lipid globules are emulsified in the aqueous phase. Alternatively the lipid globules are present in a powder and the powder is suitable for reconstitution with water or another food grade aqueous phase, preferably to provide a ready to drink formula. The lipid globules comprise a core and a surface. The core preferably comprises vegetable lipid and preferably comprises at least 90 wt.% triglycerides and more preferably essentially consists of triglycerides. Not all vegetable lipids that are present in the composition need necessarily be comprised in the core of lipid globules, but preferably a major part is, preferably more than 50% wt.%, more preferably more than 70 wt.%, even more preferably more than 85 wt.%, even more preferably more than 95 wt.%, most preferably more than 98 wt.% of the vegetable lipids that are present in the composition are comprised in the core of lipid globules. In one embodiment the core of the lipid globules comprises at least 40 wt.% triglycerides of vegetable origin, more preferably at least 50 wt.%, even more preferably at least 70 wt.% triglycerides of vegetable origin, more preferably the core of the lipid globules comprises at least 85 wt.%, more preferably at least 95 wt.% triglycerides of vegetable origin. The lipid globules in the nutritional composition in the method or use of the present invention have a volume-weighted mode diameter above 1.0 µm, preferably above 3.0 µm, more preferably 4.0 µm or above, preferably between 1.0 and 10 µm, more preferably between 2.0 and 8.0 µm, even more preferably between 3.0 and 8.0 µm, most preferably between 4.0 µm and 8.0 µm. Preferably in addition the size distribution is in such a way that at least 45 volume %, preferably at least 55 volume %, even more preferably at least 65 volume %, even more preferably at least 75 volume % has a diameter between 2 and 12 µm. More preferably at least 45 volume %, preferably at least 55 volume %, even more preferably at least 65 volume %, even more preferably at least 75 volume % has a diameter between 2 and 10 µm. Even more preferably at least 45 volume %, preferably at least 55 volume %, even more preferably at least 65 volume %, even more preferably at least 75 volume % has a diameter between 4 and 10 µm. Preferably less than 5 volume % has a diameter above 12 um.

The percentage of lipid globules is based on volume of total lipid. The mode diameter relates to the diameter which is the most present based on volume of total lipid, or the peak value in a graphic representation, having on the X-as the diameter and on the Y-as the volume (%).

The volume of the lipid globule and its size distribution can suitably be determined using a particle size analyzer such as a Mastersizer (Malvern Instruments, Malvern, UK), for example by the method described in Michalski et al, 2001, Lait 81: 787-796.

### Phospholipids

The nutritional composition to be administered in the method or use according to the present invention comprises phospholipids, preferably the nutritional composition comprises phospholipids derived from mammalian milk, preferably derived from non-human mammalian milk. Phospholipids derived from non-human mammalian milk include phospholipids isolated from milk lipid, cream lipid, cream serum lipid, butter serum lipid beta serum lipid, whey lipid, cheese lipid and/or buttermilk lipid. The buttermilk lipid is typically obtained during the manufacture of buttermilk. The butter serum lipid or beta serum lipid is typically obtained during the manufacture of anhydrous milk fat from cream or butter. Preferably the phospholipids are obtained from milk cream. The phospholipids are preferably derived from milk of cows, mares, sheep, goats, buffalos, horses and camels, most preferably from cow's milk. It is most preferred to use a lipid extract isolated from cow's milk. A suitable source of phospholipids derived from non-human mammalian milk is the fraction that can be isolated from milk called milk fat globule membrane (MFGM). Hence in one embodiment, the phospholipids to be used in the nutritional composition in the method or use according to the present invention are derived from or form part of the milk fat globule membrane (MFGM), or are provided as MFGM, preferably cow's milk MFGM.

The nutritional composition comprises 0.5 to 20 wt.% phospholipids based on total lipid, more preferably 0.5 to 10 wt.%, more preferably 1 to 10 wt.%, even more preferably 2 to 10 wt.% even more preferably 3 to 8 wt.% phospholipids based on total lipid.

The lipid globules that are present in the nutritional composition for use according to the present invention are at least partly coated on the surface with phospholipids. By 'coating' is meant that the outer surface layer of the lipid globule comprises phospholipids, whereas these phospholipids are virtually absent in the core of the lipid globule. The presence of phospholipids as a coating or outer layer of the lipid globule in the diet administered was found to advantageously promote a growth trajectory or body development that is more similar to that of human milk fed infants, the effect being particularly apparent in infants born to mothers that were overweight or obese at the time of conception. Not all phospholipids that are present in the composition need necessarily be comprised in the coating, but preferably a major part is. Preferably more than 30 wt.%, more preferably more than 50 wt.%, more preferably more than 70 wt.%, even more preferably more than 85 wt.%, most preferably more than 95 wt.% of the phospholipids that are present in the composition are comprised in the coating of lipid globules. Preferably the phospholipids comprise at least 15 wt.% sphingomyelin based on total phospholipids.

According to the present invention, the nutritional composition preferably comprises glycerophospholipids. Glycerophospholipids are a class of lipids formed from fatty acids esterified at the hydroxyl groups on carbon-1 and carbon-2 of the backbone glycerol moiety and a negatively-charged phosphate group attached to carbon-3 of the glycerol via an ester bond, and optionally a choline group (in case of phosphatidylcholine, PC), a serine group (in case of phosphatidylserine, PS), an ethanolamine group (in case of phosphatidylethanolamine, PE), an inositol group (in case of phosphatidylinositol, PI) or a glycerol group (in case of phosphatidylglycerol, PG) attached to the phosphate group. Lysophospholipids are a class of phospholipids with one fatty acyl chain. Preferably the present composition contains PC, PS, PI and/or PE, more preferably at least PC.

Preferably the nutritional composition comprises sphingomyelin. Sphingomyelins have a phosphorylcholine or phosphorylethanolamine molecule esterified to the 1-hydroxy group of a ceramide. They are classified as phospholipid as well as sphingolipid, but are not classified as a glycerophospholipid nor as a glycosphingolipid. Preferably the nutritional composition comprises 0.05 to 10 wt.% sphingomyelin based on total lipid, more preferably 0.1 to 5 wt.%, even more preferably 0.2 to 2 wt.%.

According to the present invention, the nutritional composition preferably comprises cholesterol. The nutritional composition preferably comprises at least 0.005 wt.% cholesterol based on total lipid, more preferably at least 0.01 wt.%, more preferably at least 0.02 wt.%, more preferably at least 0.05 wt.%., even more preferably at least 0.1 wt.%. Preferably the amount of cholesterol does not exceed 10 wt.% based on total lipid, more preferably does not exceed 5 wt.%, even more preferably does not exceed 1 wt.% of total lipid.

Methods for obtaining lipid globules with an increased size and coating with phospholipids are disclosed in WO 2010/0027258, WO 2010/0027259 and WO 2013/135738.

### Infant formula and follow on formula

The nutritional composition to be administered in the method or use according to the present invention is selected from an infant formula and a follow on formula. This means that the present nutrition composition is not human milk. Alternatively the term "formula" means that it concerns a composition that is artificially made or in other words that it is synthetic. Hence in one embodiment the nutritional composition is selected from an artificial infant formula and an artificial follow on formula or a synthetic infant formula and a synthetic follow on formula. In the present context, infant formula refers to nutritional compositions, artificially made, intended for infants of 0 to about 4 to 6 months of age and are intended as a substitute for human milk. Typically infant formulas are suitable to be used as sole source of nutrition. Such formulas are also known as starter formula. Formula for infants starting with at 4 to 6 months of life to 12 months of life are intended to be supplementary feedings to infants that start weaning on other foods. Such formulas are also known as follow on formulas. Infant and follow on formulas are subject to strict regulations, for example for the EU Commission Directive 2006/141/EC.

The nutritional composition preferably comprises 3 to 7 g lipid/100 kcal, preferably 4 to 6 g lipid/100 kcal, more preferably 4.5 to 5.5 g lipid/100 kcal, 1.25 to 5 g protein/100 kcal, preferably 1.35 to 4 g protein/100 kcal, more preferably 1.5 to 3 g protein/100 kcal, more preferably 1.25 to 2.5 g protein/100 kcal, more preferably 1.25 to 2.25 g/100 kcal, even more preferably 1.25 to 2.1 g protein/100 kcal and 6 to 18 g digestible carbohydrate/100 kcal, preferably 8 to 16 g digestible carbohydrate/100 kcal, more preferably 10 to 15 g digestible carbohydrate/100 kcal. The nutritional composition to be administered in the method or use according to the present invention comprises carbohydrates, protein and lipids wherein preferably the lipids provide 30 to 60 % of the total calories, the protein provides 5 to 20 % of the total calories and the carbohydrates provide 25 to 75 % of the total calories. Preferably the nutritional composition comprises 10 to 50 wt.% lipids based on dry weight of the total composition.

### Lipid

Herein LA refers to linoleic acid and/or acyl chain (18:2 n6); ALA refers to α-linolenic acid and/or acyl chain (18:3 n3); PUFA refers to polyunsaturated fatty acids and/or acyl chains; MUFA refers to monounsaturated fatty acids and/or acyl chains; LC-PUFA refers to long chain polyunsaturated fatty acids and/or acyl chains comprising at least 20 carbon atoms in the fatty acyl chain and with 2 or more unsaturated bonds; DHA refers to docosahexaenoic acid and/or acyl chain (22:6, n3); EPA refers to eicosapentaenoic acid and/or acyl chain (20:5 n3); ARA refers to arachidonic acid and/or acyl chain (20:4 n6); DPA refers to docosapentaenoic acid and/or acyl chain (22:5 n3). PA relates to palmitic acid and/or acyl chains (C16:0). Medium chain fatty acids (MCFAs) refer to fatty acids and/or acyl chains with a chain length of 6, 8 or 10 carbon atoms.

The lipid in the nutritional composition to be administered in the method or use according to the present invention preferably comprises vegetable lipids. The lipid that is present in the nutritional composition in the method or use according to the invention preferably comprises PUFAs, more preferably LC-PUFAs, as LC-PUFAs further improve the growth patterns and BMI development. The nutritional composition preferably comprises 5 to 35 wt.% PUFA, more preferably 10 to 30 wt.% PUFA, most preferably 15 to 20 wt.% PUFA, based on total lipid. In one embodiment the lipid in the nutritional composition for the method or use according to the invention comprises at least 10 wt.% polyunsaturated fatty acid based on total lipid. It is also preferred that the nutritional composition comprises MUFAs, preferably 10 to 80 wt.% MUFA, more preferably 20 to 70 wt.% MUFA, most preferably 35 to 55 wt.% MUFA, based on total lipid.

LA preferably is present in a sufficient amount in order to promote a healthy growth and development, yet in an amount as low as possible to prevent occurrence of unbalance in growth or body development. The nutritional composition therefore preferably comprises less than 20 wt.% LA based on total lipid, preferably less than 15 wt.% LA based on total lipid. Preferably, the nutritional composition comprises at least 5 wt.% LA based on total lipid, preferably the nutritional composition comprises 5 to 15 wt.% LA based on total lipid. Preferably, ALA is present in a sufficient amount to promote a healthy growth and development of the infant. The nutritional composition therefore preferably comprises at least 1.0 wt.% ALA based on total lipid. Preferably the nutritional composition comprises at least 1.5 wt.% ALA based on total lipid, more preferably at least 2.0 wt.%. Preferably the nutritional composition comprises less than 12.5 wt.% ALA, more preferably less than 10.0 wt.%, most preferably less than 5.0 wt.% ALA based on total lipid. Preferably the nutritional composition comprises 1 to 5 wt.% ALA based on total lipid. Preferably the nutritional composition comprises less than 15 wt.% linoleic acid and more than 1 wt.% alpha-linolenic acid based on total lipid. The nutritional composition comprises a weight ratio of LA/ALA from 2 to 20, more preferably from 3 to 16, more preferably from 4 to 14, more preferably from 5 to 12.

Preferably the nutritional composition comprises less than 10 wt.% short chain fatty acids based on total lipid, preferably less than 8 wt.%, preferably less than 6 wt.%, preferably less than 5 wt.%. Preferably the nutritional composition comprises at least 0.5 wt.% short chain fatty acids based on total lipid, preferably at least 0.6 wt.%, less than 8 wt.%, preferably at least 0.9 wt.%, more preferably at least 1.2 wt.%, more preferably at least 2.0 wt.%. Short chain fatty acids are fatty acids with an acyl chain of 2 to 6 carbon atoms. Preferably the nutritional composition comprises less than 10 wt.% butyric acid (acyl chain of 4 carbon atoms) based on total lipid, preferably less than 8 wt.%, preferably less than 6 wt.%, preferably less than 5 wt.%, preferably less than 4 wt.%. Preferably the nutritional composition comprises at least 0.5 wt.% butyric acid based on total lipid, preferably at least 0.6 wt.%, preferably at least 0.9 wt.%, more preferably at least 1.2 wt.%. The nutritional composition preferably comprises at least 3 wt.% MCFA based on total lipid, more preferably at least 10 wt.%, even more preferably 15 wt.%. The present composition advantageously comprises less than 50 wt.% MCFA based on total lipid, more preferably less than 30 wt.%, even more preferably less than 20 wt.%.

According to the present invention, the nutritional composition preferably comprises LC-PUFA, more preferably n-3 LC-PUFA, since n-3 LC-PUFA promote an advantageous growth trajectory. More preferably, the nutritional composition comprises EPA, DPA and/or DHA, even more preferably DHA. Since a low concentration of DHA, DPA and/or EPA is already effective and normal growth and development are important, the content of n-3 LC-PUFA in the nutritional composition, more preferably DHA, preferably does not exceed 15 wt.% of the total lipid content, preferably does not exceed 10 wt.%, even more preferably does not exceed 5 wt.%. Preferably the nutritional composition comprises at least 0.15 wt.%, preferably at least 0.35 wt.%, more preferably at least 0.75 wt.%, n-3 LC-PUFA, more preferably DHA, of the total lipid. In one embodiment, the present composition comprises at least 0.15 wt.% n-3 LC-PUFA based on total lipid selected from the group consisting of DHA, EPA, and DPA, more preferably DHA.

As the group of n-6 fatty acids, especially arachidonic acid (ARA) and LA as its precursor, counteracts the group of n-3 fatty acids, especially DHA and EPA and ALA as their precursor, the nutritional composition comprises relatively low amounts of ARA. The n-6 LC-PUFA, more preferably ARA, content preferably does not exceed 5 wt.%, more preferably does not exceed 2.0 wt.%, more preferably does not exceed 0.75 wt.%, even more preferably does not exceed 0.5 wt.%, based on total lipid. As the presence of ARA is not necessary for promoting a growth trajectory or BMI development similar to that of human milk fed infants, ARA may also be absent.

### Palmitic acid at sn-2 position of triglyceride

The lipid in the nutritional composition to be administered in the method or use according to the present invention comprises triglycerides. Triglycerides comprise a glyceride molecule to which, via ester bonds, three fatty acid residues are attached, which may be the same or different, and which are generally chosen from saturated and unsaturated fatty acids containing 6 to 26 carbon atoms, including but not limited to LA, ALA, oleic acid (C18:1), PA and/or stearic acid (C18:0). Preferably the nutritional composition comprises at least 70 wt.%, more preferably at least 80 wt.%, more preferably at least 85 wt.% triglycerides, even more preferably at least 90 wt.% triglycerides based on total lipids. The fatty acid triglycerides may differ in the fatty acid residues that are present and/or in the respective position(s) of the fatty acid residues, e.g. in the sn-1, -2 and/or -3 position. The triglycerides used in the nutritional composition are chosen such that the amount of PA residues that are present in the triglycerides are 10 wt.% or more based on total fatty acid present in the triglycerides, preferably more than 15 wt.%. The nutritional composition in the method or use according to the invention comprises lipid that comprises at least 10 wt.% palmitic acid based on total lipid, and wherein at least 15 % of the palmitic acid is present at the sn-2 position of the triglycerides, preferably at least 30 % of the palmitic acid is present at the sn-2 position of the triglycerides. Preferably the amount of PA residues that are present in the triglycerides are below 30 wt.%, more preferably in the range of 16 to 24 %. Preferably the triglycerides used in the nutritional composition are chosen such that of the total PA residues present in the triglyceride at least 20 %, more preferably at least 30 %, even more preferably at least 35 %, and most preferably at least 40 % are in the sn-2 or beta position of the triglyceride.

Suitable triglycerides for the nutritional composition in the method or use according to the invention are commercially available, e.g. from Loders Croklaan under the name Betapol^{™} and/or can be prepared in a manner known per se, for instance as described in EP 0 698 078 and/or EP 0 758 846. Another suitable source is InFat^{™} of Enzymotec. In case these lipids are obtained by trans- or interesterification of vegetable triglycerides, these sources are in the context of the present invention regarded as vegetable lipids. Preferably the amount of the triglyceride with increased amount of palmitic acid residues on the sn-2 position of a triglyceride molecule that is comprised in the lipid fraction of the composition that is to be administered according to the present method or use, ranges from 10 to 100 wt.%, preferably from 20 to 100 wt.%, more preferably from 20 to 80 wt.%, even more preferably from 50 to 80 wt.%.

A preferred source for triglycerides having palmitic acid at the sn-2 or beta position of the triglyceride is non-human animal lipid, more preferably non-human mammalian milk lipid, even more preferably cow's milk lipid. Preferably non-human mammalian milk lipid, in particular cow's milk lipid, is preferably derived from the group consisting of butter, butter fat, butter oil, and anhydrous milk fat, more preferably anhydrous milk fat and butter oil. Preferably the source of the milk lipid is in a homogenous fat phase, such as butter oil or anhydrous milk fat, and not in the form of oil in water emulsion such as cream, since the lipid globules of the present nutritional composition can be more easily prepared when in a homogenous fat phase. Preferably the amount of milk lipid ranges from 10 to 100 wt.% based on total lipid, preferably from 10 to 80 wt.% based on total lipid, more preferably from 10 to 70 wt.%, more preferably from 20 to 80 wt.%, more preferably from 15 to 60 wt.%, more preferably from 20 to 60 wt.%, even more preferably from 25 to 50 wt.% based on total lipid.

In an alternative embodiment, the present invention concerns a method for improving the postnatal growth trajectory or body development in an infant that is born to a mother that is overweight or obese at the time of conception (pre-pregnancy) of the infant, said method comprising feeding said infant a nutritional composition selected from an infant formula and a follow-on formula comprising carbohydrates, protein and lipid,
i) wherein the lipid comprises linoleic acid and alpha-linolenic acid in a weight ratio of 2 to 20, and
ii) the lipid comprises 10 to 70 wt.% based on total lipid of non-human mammalian milk lipid derived from the group consisting of butter, butter fat, butter oil, and anhydrous milk fat, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

In other words, in an alternative embodiment, the present invention concerns a nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid comprises 10 to 70 wt.% based on total lipid of non-human mammalian milk lipid derived from the group consisting of butter, butter fat, butter oil, and anhydrous milk fat, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids,
for use in improving the postnatal growth trajectory or body development in an infant that is born to a mother that is overweight or obese at the time of conception of the infant.

The invention in an alternative embodiment can also be worded as the use of lipid globules in the manufacture of a nutritional composition selected from an infant formula and a follow on formula for use in improving the postnatal growth trajectory or body development in an infant that is born to a mother that is overweight or obese at the time of conception of the infant, wherein the nutritional composition comprises carbohydrates, protein and lipid, wherein,
i) the lipid comprises linoleic acid and alpha-linolenic acid in a weight ratio of 2 to 20,
ii) the lipid comprises 10 to 70 wt.% based on total lipid of non-human mammalian milk lipid derived from the group consisting of butter, butter fat, butter oil, and anhydrous milk fat, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

The present invention concerns in an alternative embodiment a method for preventing or reducing the risk of an unbalanced postnatal growth trajectory or body development in an infant at risk of having an unbalanced postnatal growth trajectory or body development, said infant being born to a mother that is overweight or obese at the time of conception of the infant, said method comprising feeding said infant a nutritional composition selected from an infant formula and a follow on formula comprising carbohydrates, protein and lipid,
i) wherein the lipid comprises linoleic acid and alpha-linolenic acid in a weight ratio of 2 to 20,
ii) the lipid comprises 10 to 70 wt.% based on total lipid of non-human mammalian milk lipid derived from the group consisting of butter, butter fat, butter oil, and anhydrous milk fat, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

The invention in an alternative embodiment also concerns a nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid 10 to 70 wt.% based on total lipid of non-human mammalian milk lipid derived from the group consisting of butter, butter fat, butter oil, and anhydrous milk fat, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids,
for use in preventing or reducing the risk of an unbalanced postnatal growth trajectory or body development in an infant at risk of having an unbalanced postnatal growth trajectory or body development, said infant being born to a mother that is overweight or obese at the time of conception of the infant.

The invention in an alternative embodiment can also be worded as the use of lipid globules in the manufacture of a nutritional composition selected from an infant formula and a follow on formula for use in preventing or reducing the risk of an unbalanced postnatal growth trajectory or body development in an infant at risk of having an unbalanced postnatal growth trajectory or body development, said infant being born to a mother that is overweight or obese at the time of conception of the infant, wherein the nutritional composition comprises carbohydrates, protein and lipid, wherein,
i) the lipid comprises linoleic acid and alpha-linolenic acid in a weight ratio of 2 to 20,
ii) the lipid comprises 10 to 70 wt.% based on total lipid of non-human mammalian milk lipid derived from the group consisting of butter, butter fat, butter oil, and anhydrous milk fat, and wherein the nutritional composition comprises lipid globules that have

a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

Non-human mammalian milk lipid in the present invention refers to all lipid components of milk, as produced by the mammalians, such as the cow, and is found in commercial milk and milk-derived products.

Butter in the present invention is a water-in-oil emulsion comprised of over 80 wt.% milk lipid.

Butterfat in the present invention relates to all of the lipid components in milk that are separable by churning, in other words, present in butter.

Anhydrous milk fat (AMF) is a term known in the art and relates to extracted milk lipid. Typically AMF comprises more than 99 wt.% lipid based on total weight. It can be prepared from extracting milk lipid from cream or butter. Anhydrous butter oil in the present invention is synonymous with AMF.

Butter oil also is a term known in the art. It typically relates to a milk lipid extract with more than 98 wt.% lipid and typically is a precursor in the process of preparing anhydrous milk fat or anhydrous butter oil.

Preferably the non-human mammalian milk comprises at least 70 wt.% triglycerides, more preferably at least 90 wt.%, more preferably at least 97 wt.%.

Preferably the non-human mammalian milk lipid is anhydrous milk fat or butter oil. Such milk fat lipid sources are high in triglyceride levels. Furthermore these lipid sources are in the form of a continuous fat phase or a water-in-oil emulsion form and not in the form of oil in water emulsion such as cream, since the lipid globules of the present nutritional composition can be more easily prepared when in a homogenous fat phase.

Preferably the amount of non-human mammalian milk lipid ranges from 10 to 70 wt.% based on total lipid, preferably ranges from 20 to 70 wt.%, more preferably from 15 to 60 wt.%, more preferably from 20 to 60 wt.%, even more preferably from 25 to 50 wt.% based on total lipid.

### Protein

The nutritional composition comprises proteins, preferably in the amounts specified above. Preferably the protein provides 5 to 9% of the total calories of the nutritional composition. The source of the protein should be selected in such a way that the minimum requirements for essential amino acid content are met and satisfactory growth is ensured. Hence protein sources based on cows' milk proteins such as whey, casein and mixtures thereof and proteins based on soy, potato or pea are preferred. In case whey proteins are used, the protein source is preferably based on acid whey or sweet whey, whey protein isolate or mixtures thereof and may include α-lactalbumin and β-lactoglobulin. More preferably, the protein source is based on acid whey or sweet whey from which caseino-glyco-macropeptide (CGMP) has been removed. Preferably the composition comprises at least 3 wt.% casein based on dry weight. Preferably the casein is intact and/or non-hydrolyzed. For the present invention protein includes peptides and free amino acids.

### Digestible carbohydrates

The nutritional composition comprises digestible carbohydrate, preferably in the amounts specified above. Preferred digestible carbohydrate sources are lactose, glucose, sucrose, fructose, galactose, maltose, starch and maltodextrin. Lactose is the main digestible carbohydrate present in human milk. Lactose advantageously has a low glycemic index. The nutritional composition preferably comprises lactose. The nutritional composition preferably comprises digestible carbohydrate, wherein at least 35 wt.%, more preferably at least 50 wt.%, more preferably at least 75 wt.%, even more preferably at least 90 wt.%, most preferably at least 95 wt.% of the digestible carbohydrate is lactose. Based on dry weight the present composition preferably comprises at least 25 wt.% lactose, preferably at least 40 wt.%.

### Non digestible carbohydrates

In one embodiment the nutritional composition comprises non-digestible oligosaccharides. Preferably the nutritional composition comprises non-digestible oligosaccharides with a degree of polymerization (DP) between 2 and 250, more preferably 3 and 60.

Preferably the present composition comprises fructo-oligosaccharides, inulin and/or galacto-oligosaccharides, more preferably galacto-oligosaccharides, most preferably transgalacto-oligosaccharides. In a preferred embodiment the composition comprises a mixture of transgalacto-oligosaccharides and fructo-oligosaccharides or inulin. Suitable non-digestible oligosaccharides are for example Vivinal^{®}GOS (FrieslandCampina DOMO), RaftilinHP^{®} or Raftilose^{®} (Orafti).

Preferably, the nutritional composition comprises of 80 mg to 2 g non-digestible oligosaccharides per 100 ml, more preferably 150 mg to 1.50 g, even more preferably 300 mg to 1 g per 100 ml. Based on dry weight, the nutritional composition preferably comprises 0.25 wt.% to 20 wt.%, more preferably 0.5 wt.% to 10 wt.%, even more preferably 1.5 wt.% to 7.5 wt.%.

### Application

In the method or use according to the present invention, a nutritional composition is administered to an infant or is used in an infant. In the context of the present invention an infant has an age up to 12 months. Preferably the nutritional composition is administered to or is used in a term born infant. A term infant means an infant born art a gestational age of 37 to 42 weeks. Preferably the nutritional composition is administered to or is used in a healthy infant. Preferably the nutritional composition is used at least during the first 2 months of life, preferably at least during the first 3 months of life of the infant, more preferably at least during the first 4 months of life of the infant. Preferably the nutritional composition is administered to an infant with an age below 6 months, more preferably below 4 months of age.

According to the present invention the postnatal growth trajectory or body development in an infant that is born to a mother that is overweight or obese at the time of conception of the infant is improved. Preferably the growth trajectory or body development is the trajectory or development of body mass index (BMI), preferably the trajectory or development body mass index (BMI) in the first 4 months of life of the infant, preferably the trajectory or development body mass index (BMI) in the first 12 months of life of the infant. In one embodiment, improving the postnatal growth trajectory or body development of the infant is reducing the increase of BMI of the infant. Preferably the improvement is compared to the postnatal growth trajectory or body development in an infant born to a mother that is overweight or obese at the time of conception of the infant fed infant formula or follow on formula comprising carbohydrates, protein and lipid, and comprising at least 10 wt.% palmitic acid based on total lipid, yet with less than 15 % of the palmitic acid residues being in the sn-2 position of triglycerides and not comprising phospholipids and having lipid globules with a mode diameter, based on volume, of about 0.5 µm. Thus in such an infant or follow on formula the volume % of lipid globules with a mode between 2 and 12 µm is below 10 vol.%. Preferably for a proper comparison the age of the infant and time period over which the nutritional composition is administered correspond.

According to the present invention an unbalanced postnatal growth trajectory or body development in an infant at risk of having an unbalanced postnatal growth trajectory or body development, said infant being born to a mother that is overweight or obese at the time of conception of the infant is prevented or the risk thereof is reduced. Preferably the growth trajectory or body development is the trajectory or development of body mass index (BMI), preferably the trajectory or development body mass index (BMI) in the first 4 months of life of the infant, preferably the trajectory or development body mass index (BMI) in the first 12 months of life of the infant. Preferably the prevention or reduction of the risk is compared to the postnatal growth trajectory or body development in an infant born to a mother that is overweight or obese at the time of conception of the infant fed infant formula or follow on formula comprising carbohydrates, protein and lipid, and comprising at least 10 wt.% palmitic acid based on total lipid, yet with less than 15 % of the palmitic acid residues being in the sn-2 position of triglycerides and not comprising phospholipids and having lipid globules with a mode diameter, based on volume, of about 0.5 µm. Thus in such an infant or follow on formula the volume % of lipid globules with a mode between 2 and 12 µm is below 10 vol.%. Preferably for a proper comparison the age of the infant and time period over which the nutritional composition is administered correspond.

Preferably the infant formula or follow on formula, when ready to drink has an energy density of 60 kcal to 75 kcal/100 ml, more preferably 60 to 70 kcal/100 ml. This density ensures an optimal balance between hydration and caloric intake.

In one embodiment, the infant formula or follow on formula is a powder. Suitably, the infant formula or follow on formula is in a powdered form, which can be reconstituted with water or other food grade aqueous liquid, to form a ready-to drink liquid, or is in a liquid concentrate form that should be diluted with water to a ready-to-drink liquid. It was found that lipid globules maintained their size and coating when reconstituted.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Test and Control formula

### Diet 1: Control infant formula (Control formula)

The Control formula comprised per 100 ml ready to drink formula 66 kcal, 1.3 g protein (whey protein and casein in a 6/4 w/w ratio), 7.3 g digestible carbohydrates (mainly lactose), 3.4 g lipid and 0.8 g short chain galacto-oligosaccharides (source Vivinal^{®}GOS) and long chain fructo-oligosaccharides (source RaftilinHP^{®}) in a 9/1 w/w ratio, and minerals, vitamins, trace elements and other micronutrients in compliance with directives for infant formula. The formula is provided as a powder with the instruction to reconstitute with water, about 13.6 g powder is to be reconstituted to obtain a 100 ml ready to drink infant formula.

The lipid component comprised mainly vegetable lipid (blend of palm oil, low erucic acid rape seed oil, coconut oil, high oleic sunflower oil, sunflower oil, a small amount of soy lecithin (0.13 wt.%) and about 1.5 wt.% of an LC-PUFA premix (fish oil and microbial oil).

The lipid component was present in the form of lipid globules and the lipid globules had a mode diameter, based on volume, of about 0.5 µm, and the volume % of lipid globules with a mode between 2 and 12 µm was below 10 vol.%.

### Diet 2: Test infant formula (Test formula)

The Test formula was an infant formula similar to Diet 1, except for the following differences:
The lipid globules in the Test formula had a mode diameter, based on volume, of about 5.6 µm, and the volume % of lipid globules with a mode between 2 and 12 µm was above 45 vol.%.

The lipid component consisted of about 51 wt.% vegetable lipid (blend of low erucic acid rape seed oil, coconut oil, high oleic sunflower oil, sunflower oil), about 44 wt.% bovine anhydrous milk fat, 1.5 wt.% LC-PUFA containing oil (fish oil and microbial oil), 0.13 wt.% soy lecithin, about 3.6 wt.% milk lipid derived from buttermilk rich in milk phospholipids or milk fat globule membranes (milk phospholipids were about 1.5 wt.% based on total lipid).

The fatty acid composition was very similar between diet 1 and 2, in saturated, mono unsaturated and poly unsaturated acids, and in n3 and n6 PUFA content. The amount of palmitic acid was 18.4 wt.% and 17.7 wt.% (based on total lipid) for diet 1 and 2, respectively. For diet 2 about 36 wt.% of the palmitic acid residues was in the sn2 position, while for diet 1 this was about 13 wt.%. The amount of C4:0 (butyric acid) was 0.10 wt.% in diet 1 and 1.39 wt.% in diet 2, C6:0 (caproic acid) was 0.24 wt.% in diet 1 and 0.98 wt.% in diet 2. The wt.% are based on total lipid in the infant formula, unless indicated otherwise.

### Example 2: Study protocol and study population

After parent(s)/legal guardian(s) have signed informed consent, exclusively formula fed infants, eligible for participation, were randomised to receive either the Test Formula (Diet 2) or the Control formula (Diet 1) for a double-blind period of maximally 17 weeks (depending on their age at study entry). Exclusively breastfed infants participated in the reference group and had the same visit schedule and study assessments as the randomised infants.

At the first visit, baseline and birth data were collected, as well as maternal pre-pregnancy weight, race and length to derive the BMI (kg/m²), and the study product and diaries were provided to the parent(s). Further study visits were conducted at 5, 8, 13, 17 and 52 weeks of age. Information and anthropometrical measurements were collected during the visits. During the visit at 52 weeks, anthropometrical measurements were collected.

In total 4 countries with 17 sites participated, and in total 313 subjects were enrolled; 6 sites in the Netherlands (121 subjects), 3 sites in France (13 subjects), 7 sites in Belgium (158 subjects), and 1 site in Singapore (21 subjects). Of the total of 313 enrolled subjects, 223 were randomised and 88 were included in the breastfed reference group, 2 subjects were screen failures and were consequently not randomised.

The All-Subjects-Treated (AST) data set consisted of all subjects randomised (ASR, n= 223) who received at least some study product. Subjects (n=8), with sufficient evidence that no study product was consumed, were considered as non-treated, and were not included in the AST group (n= 215).

The ITT data set consisted of all subjects from the ASR group (ITT = ASR). Results from the ITT analysis reflect the effects on the targeted population in a real clinical situation / estimates the effect (effectiveness) of the treatment policy. Subjects' data were analysed 'as randomised' (n=223).

A Per-Protocol (PP) analysis restricts the analysis to the subjects who fulfil the protocol in the terms of eligibility, interventions, instructions/restrictions and outcome assessment. The PP data set consisted of all subjects and/or subjects' visits from the ITT data set without any major protocol deviations. Thus, the PP dataset was not limited to subjects who completed the study, and the number of subjects per visits varies. Results from the PP analysis estimate the effect (efficacy) of the treatment. Subjects' data were analysed 'as treated'.

The following rules have been applied for exclusion of subjects from the PP data set: Age at baseline (= visit 1) >35 days, birth weight missing or is <9.96th or >90.04th percentile (based on WHO Child growth standard references), head circumference at inclusion is outside ± 2.04 SD percentile (based on WHO Child growth standard references), not having at least one valid post-baseline visit. Study product consumption started ≥6 days after baseline, having received a different study product as his/her twin sibling, no study product was consumed, relevant medical history, i.e. illnesses/conditions as identified by the Medical Monitor. The following rules have been applied for exclusion of distinct visits from the PP data set: Any visit >3 days after stop of study product intake with the exception of the visit at 52 weeks, regardless if stop was temporarily or not, any visit >3 days after start of other formula feeding, any visit >3 days after start of solid feeding. 49 randomised subjects plus certain visits were excluded.

For the non-randomised breastfed reference group, data sets corresponding to the ITT and PP populations of the randomised infants have been defined, too. Correspondingly to the ITT data set, a full breastfed group (FBF) has been defined, no breastfed subjects were excluded. Correspondingly to the PP dataset, a Protocol Compliant Breastfed Reference (PCBF) data set has been defined, applying the relevant rules as defined for the PP dataset. The following rules have been applied for exclusion of subjects from the PCBF data set: Age at baseline >35 days, birth weight missing or is <9.96th or >90.04th percentile (based on WHO Child growth standard references), head circumference at inclusion is outside ± 2.04 SD (based on WHO Child growth standard references), not having at least one valid post-baseline visit, or relevant medical history, i.e. illnesses/conditions as identified by the Medical Monitor. The following rules have been applied for exclusion of distinct visits from the PCBF data set: Any visit >3 days after stop of breastfeeding, in case stop of breastfeeding occurred before 13 weeks of age, any visit >3 days after start of other formula feeding, in case start of other formula feeding occurred before 13 weeks of age, any visit >3 days after start of solid feeding, in case start of other solid feeding occurred before visit 4. 11 breastfed subjects plus certain visits were excluded.

Subjects (either randomised or breastfed) who were included in PP/PCBF dataset up and including visit at 17 weeks of age and participated in the optional extension, were included in PP dataset at visit at 52 weeks.

There were no statistical significant differences between the intervention groups within the different datasets (PP, ITT) on the stratification factors sex, age at baseline (≤14 days/ >14 days), regions (Europe vs. Asia). There was no difference in the study duration between the intervention groups.

Maternal pre-pregnancy weight categories are defined according to WHO criteria (Obesity: Preventing and managing the global epidemic. Report of a WHO Consultation on Obesity. Geneva: World Health Organisation.) as follows: Underweight with a BMI below 18.5 kg/m², normal for a BMI between 18.5 kg/m² and 25 kg/m², overweight or obese for a BMI equal or above 25 kg/m². The WHO adapted the recommendation for maternal weight categories for mothers of Asian origin (WHO/IOTF/IASO 2000, WHO Expert consultation 2004, Lancet 363: 157-163) as follows: underweight with a BMI below 18.5, normal weight with a BMI between 18.5 and 23 and overweight or obese with a BMI equal or above 23.

Table 1 provides a tabular overview of the PP population, showing the number and percentage of subjects by maternal pre-pregnancy weight category, for the Test formula, Control formula and breastfed group.

**Table 1. Study population statistics (n (%)) for the different maternal pre-pregnancy weight categories of the PP population**

| **Maternal pre-pregnancy BMI** | **Control (N=83)** | **Test (N=91)** | **Breastfed (N=77)** |
|---|---|---|---|
| **Underweight** | 2 (2.44%) | 7 (7.69%) | 2 (2.6%) |
| **Normal** | 58 (70.73%) | 45 (49.45%) | 42 (54.55%) |
| **Overweight** / **Obese** | 22 (26.83%) | 39 (42.86%) | 33 (42.86%) |

Table 2 provides a tabular overview of the ITT population, showing the number and percentage of subjects by maternal pre-pregnancy weight category, for the Test formula, Control formula and breastfed group.

**Table 2. Study population statistics (n (%)) for the different maternal pre-pregnancy weight categories of the ITT population**

| **Maternal pre-pregnancy BMI** | **Control (n=108)** | **Test (n=115)** | **Breastfed (n=88)** |
|---|---|---|---|
| **Underweight** | 3 (2.80%) | 10 (8.77%) | 3 (3.41 %) |
| **Normal** | 74 (69.16%) | 60 (52.63%) | 44 (50.00%) |
| **Overweight / Obese** | 30 (28.04%) | 44 (38.60%) | 41 (46.69%) |

Table 3 provides a tabular overview of the PP population, showing at baseline, 4 months and 1 year of age the number of subjects and their mean (SD) BMI by maternal pre-pregnancy weight category, for the Test formula, Control formula and breastfed group. Table 4 provides the same information for the ITT population.

**Table 3. Study population statistics for the number of subjects (n) and mean BMI (SD) at different time points for the maternal pre-pregnancy weight categories of the PP population**

| Baseline | Maternal weight category | | Test (n=91) | Control (n=83) | Breastfed (n=77) |
|---|---|---|---|---|---|
| | Underweight | n | 7 | 2 | 2 |
| | | Mean (SD) | 12.7 (1.28) | 12.9 (0.23) | 12.5 (1.82) |
| | Normal | n | 45 | 58 | 42 |
| | | Mean (SD) | 13.2 (0.96) | 13.1 (1.06) | 14.0 (1.49) |
| | Overweight/Obese | n | 39 | 22 | 33 |
| | | Mean (SD) | 13.1 (1.25) | 13.1 (1.24) | 14.0 (1.31) |
| 4 months | Underweight | n | 4 | 1 | 2 |
| | | Mean (SD) | 17.0 (0.59) | 16.8 | 16.3 (0.17) |
| | Normal | n | 35 | 42 | 39 |
| | | Mean (SD) | 16.6 (1.12) | 16.7 (1.26) | 16.6 (1.63) |
| | Overweight/Obese | n | 31 | 15 | 24 |
| | | Mean (SD) | 16.3 (1.32) | 17.2 (0.78) | 16.8 (1.64) |
| 1 year | Underweight | n | 3 | - | 2 |
| | | Mean (SD) | 16.9 (1.16) | | 16.5 (0.96) |
| | Normal | n | 25 | 26 | 31 |
| | | Mean (SD) | 16.4 (1.33) | 17.4 (1.37) | 16.7 (0.92) |
| | Overweight/Obese | n | 25 | 13 | 19 |
| | | Mean (SD) | 16.7 (1.09) | 17.6 (1.25) | 16.6 (1.37) |

**Table 4. Study population statistics for the number of subjects (n) and mean BMI (SD) at different time points for the maternal pre-pregnancy weight categories of the ITT population**

| Baseline | Maternal weight category | | Test (n=115) | Control (n=108) | Breastfed (n=88) |
|---|---|---|---|---|---|
| | Underweight | n | 10 | 3 | 3 |
| | | Mean (SD) | 12.7 (1.14) | 12.8 (0.26) | 13.4 (1.99) |
| | Normal | n | 60 | 74 | 44 |
| | | Mean (SD) | 13.2 (1.10) | 13.0 (1.04) | 14.0 (1.50) |
| | Overweight/Obese | n | 44 | 29 | 41 |
| | | Mean (SD) | 13.2 (1.20) | 13.1 (1.28) | 13.8 (1.39) |
| 4 months | Underweight | n | 5 | 2 | 2 |
| | | Mean (SD) | 16.7 (0.78) | 16.7 (0.10) | 16.3 (0.17) |
| | Normal | n | 45 | 57 | 40 |
| | | Mean (SD) | 16.8 (1.26) | 16.6 (1.17) | 16.6 (1.61) |
| | Overweight/Obese | n | 36 | 22 | 27 |
| | | Mean (SD) | 16.3 (1.35) | 17.0 (1.00) | 16.7 (1.56) |
| 1 year | Underweight | n | 4 | 1 | 2 |
| | | Mean (SD) | 16.5 (1.33) | 16.7 | 16.5 (0.96) |
| | Normal | n | 30 | 38 | 32 |
| | | Mean (SD) | 16.7 (1.50) | 17.4 (1.32) | 16.7 (0.90) |
| | Overweight/Obese | n | 29 | 19 | 21 |
| | | Mean (SD) | 16.7 (1.13) | 17.5 (1.13) | 16.7 (1.37) |

At baseline, the BMI (kg/m²) of the infants born from mothers having a normal weight or born from mothers that were overweight/obese before pregnancy were comparable. The BMI of the randomised infants was also comparable to the breastfed reference group in the PP and ITT population. At 1 year of age the BMI of the infants born from mothers having a normal weight or that were overweight/obese before pregnancy and that received the Test formula is closer to the BMI of the breastfed reference group, compared to the infants that received the Control formula in both the PP and ITT population. Overall it is apparent that the development of the BMI in the infants receiving the Test formula is more comparable to the BMI development of infants that are breast fed.

From baseline to 4 months and to 1 year of age, the difference in BMI gain (Test minus Control) for the group of infants born from mothers with normal weight and the group of infants born from overweight/obese mothers was calculated using a Parametric Growth Curve (PGC) analysis, considering the overall study period (baseline until 1 year) and correcting for stratification factors as described herein before. All statistical analyses were performed by Nutricia Research Utrecht using SAS^{®} (SAS Enterprise Guide 4.3 or higher) for Windows, SAS Institute Inc., Cary, NC. This approach assumes a parametric function of time (i.e. age of subject) and thus, describes the development of growth parameters (i.e. BMI) over time by a second order polynomial function. It does not require the study subjects to be measured at the same set of time points. The resulting parameters are compared to assess differences between the curves.

**Table 5: Differences in BMI gain (kg/m²) between the Test and the Control group for the maternal pre-pregnancy categories, for the PP study population.**

| | **Age interval** | **Maternal pre-pregnancy weight** | **LS mean (SE)** | **P-value** |
|---|---|---|---|---|
| Test minus Control | Baseline to 4 months | Normal | -0.272 (0.294) | 0.356 |
| | | Overweight/Obese | -0.980 (0.394) | 0.014 |
| | Baseline to 1 year | Normal | -0.935 (0.358) | 0.010 |
| | | Overweight / Obese | -1.200 (0.455) | 0.009 |

**Table 6: Differences in BMI gain (kg/m2) between the Test and the Control group for the maternal pre-pregnancy categories, for the ITT study population.**

| | **Age interval** | **Maternal pre-pregnancy weight** | **LS mean (SE)** | **P-value** |
|---|---|---|---|---|
| Test minus Control | Baseline to 4 months | Normal | -0.088 (0.257) | 0.733 |
| | | Overweight/Obese | -0.717 (0.346) | 0.040 |
| | Baseline to 1 year | Normal | -0.909 (0.304) | 0.003 |
| | | Overweight / Obese | -0.842 (0.386) | 0.031 |

Tables 5 and 6 (ITT and PP population) show the LS mean estimate for the difference between groups (Test minus Control) in BMI gain per age interval and maternal pre-pregnancy category. Infants that received the Control formula have a higher increase in BMI when compared to infants receiving Test formula, which was also statistically significantly higher in all age intervals and weight categories except for the interval from baseline till 4months in the infants born from a normal weight mother. Infants with overweight/obese mother who received the Control formula had a statistically significant higher BMI gain than infants receiving the Test formula, surprisingly this difference in BMI increase was 3 fold higher in the period from baseline until 4 months in the PP and even almost 8 times higher in the ITT population.

In the group of infants born from an overweight or obese mother a significant difference in BMI gain between Test formula and Control formula fed infants from baseline to 4 months and from baseline to 1 year of age is apparent. Between baseline and 4 months of age the infants born from an overweight or obese mother fed the Test formula had a significantly lower BMI gain than the infants born from an overweight or obese mother fed the Control formula (p=0.014 in the PP population, p = 0.040 in the ITT study). In infants born from normal weight mothers the difference in BMI gain between Test formula and Control formula is not apparent between baseline and 4 months of age, yet between baseline and 1 year of age the group of infant born from normal weight mothers and fed the Test formula have a significantly lower BMI than the infants fed the Control diet (p=0.010 in the PP population, p=0.003 in the ITT population). The effect of the Test diet on BMI gain is thus stronger and earlier (at 4 months) apparent in the infants born from overweight and obese mothers.

These results are indicative for a stronger effect of the Test formula, when fed to infants born from overweight or obese mothers, on promoting a postnatal growth trajectory or body development in an infant towards a more beneficial growth trajectory or body development, which is more similar to the growth trajectory or body development observed in infants which are breastfed compared to the effect it has on infants born from mothers with a normal weight. Infants born from overweight or obese mothers benefit to a larger extent from the nutritional intervention compared to infants born from mothers with a normal weight for developing a desired growth trajectory or body development. Hence an improvement of the postnatal growth trajectory or body development is achieved in infants born to an overweight or obese mother. Also these results are indicative for an effect of the Test formula for infants, when fed to infants born from overweight or obese mothers, for preventing or reducing the risk of an unbalanced postnatal growth trajectory or body development in such an infant being at risk of having an unbalanced postnatal growth trajectory or body development. In particular the improvement of the postnatal growth trajectory or body development or prevention or reduction unbalanced postnatal growth trajectory or body development are found when compared to the growth trajectory or body development in infants fed infant formula of follow on formula comprising lipid globules of about 0.5 µm and that do not have a coating of phospholipids and comprising at least 10 wt.% palmitic acid based on total lipid, and at less than 15 wt.% of the palmitic acid is esterified to the sn-2 position of a triglyceride based on total palmitic acid.

## Claims

1. A method for non-therapeutic improvement of the postnatal growth trajectory or body development in an infant that is born to a mother that is overweight or obese at the time of conception of the infant by administration of a nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have
a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids.

2. A nutritional composition selected from an infant formula and a follow on formula comprising carbohydrates, protein and lipid, wherein
i) the lipid comprises linoleic acid and alpha-linolenic acid in a weight ratio of 2 to 20,
ii) the lipid comprises at least 10 wt.% palmitic acid based on total lipid, and at least 15 wt.% of this palmitic acid is esterified to the sn-2 position of a triglyceride based on total palmitic acid, and wherein the nutritional composition comprises lipid globules that have
a) a mode diameter, based on volume of at least 1.0 µm and/or a diameter of 2 to 12 µm in an amount of at least 45 volume % based on total lipid and
b) the lipid globules are at least partly coated on the surface with phospholipids, the amount of phospholipids present in the nutritional composition being from 0.5 to 20 wt.% phospholipids based on total lipids,
for use in preventing or reducing the risk of an unbalanced postnatal growth trajectory or body development in an infant at risk of having an unbalanced postnatal growth trajectory or body development, said infant being born to a mother that is overweight or obese at the time of conception of the infant.

3. The method for non-therapeutic improvement of the postnatal growth trajectory or body development according to claim 1, wherein the improvement is compared to the postnatal growth trajectory or body development in an infant born to a mother that is overweight or obese at the time of conception of the infant fed infant formula or follow on formula comprising carbohydrates, protein and lipid, and comprising at least 10 wt.% palmitic acid based on total lipid, yet with less than 15 % of the palmitic acid residues being in the sn-2 position of triglycerides and not comprising phospholipids and having lipid globules with a mode diameter, based on volume, of about 0.5 µm.

4. The nutritional composition for use according to claim 2, wherein the prevention or reduction of the risk is compared to the postnatal growth trajectory or body development in an infant born to a mother that is overweight or obese at the time of conception of the infant fed infant formula or follow on formula comprising carbohydrates, protein and lipid, and comprising at least 10 wt.% palmitic acid based on total lipid, yet with less than 15 % of the palmitic acid residues being in the sn-2 position of triglycerides and not comprising phospholipids and having lipid globules with a mode diameter, based on volume, of about 0.5 µm.

5. The nutritional composition for use or method for non-therapeutic improvement of the postnatal growth trajectory or body development according to any of the preceding claims, wherein the nutritional composition comprises less than 15 wt.% linoleic acid and more than 1 wt.% alpha-linolenic acid based on total lipid.

6. The nutritional composition for use or method for non-therapeutic improvement of the postnatal growth trajectory or body development according to any one of the preceding claims, wherein the growth trajectory or body development is the trajectory or development of body mass index (BMI).

7. The nutritional composition for use or method for non-therapeutic improvement of the postnatal growth trajectory or body development according to any one of the preceding claims, wherein the use is for reducing the increase of BMI of the infant.

8. The nutritional composition for use or method for non-therapeutic improvement of the postnatal growth trajectory or body development according to any one of the preceding claims, wherein the growth trajectory or body development is the trajectory or development of body mass index (BMI) in the first 12 months of life of the infant.

9. The nutritional composition for use or method for non-therapeutic improvement of the postnatal growth trajectory or body development according to any one of the preceding claims, wherein the infant is a term born infant.

10. The nutritional composition for use or method for non-therapeutic improvement of the postnatal growth trajectory or body development according to any one of the preceding claims, wherein the phospholipids comprise at least 15 wt.% sphingomyelin based on total phospholipids.

11. The nutritional composition for use or method for non-therapeutic improvement of the postnatal growth trajectory or body development according to any one of the preceding claims, wherein the nutritional composition comprises phospholipids that are derived from or form part of the milk fat globule membrane (MFGM), or are provided as MFGM, preferably cow's milk MFGM.

12. The nutritional composition for use or method for non-therapeutic improvement of the postnatal growth trajectory or body development according to any one of the preceding claims, wherein the nutritional composition comprises 0.5 to 20 wt.% phospholipids derived from mammalian milk based on total lipid.

13. The nutritional composition for use or method for non-therapeutic improvement of the postnatal growth trajectory or body development according to any one of the preceding claims, wherein at least 30 wt.% of the palmitic acid is esterified to the sn-2 position of a triglyceride based on total palmitic acid.

14. The nutritional composition for use or method for non-therapeutic improvement of the postnatal growth trajectory or body development according to any one of the preceding claims, wherein the lipid globules comprise at least 40 wt.% triglycerides of vegetable origin.

15. The nutritional composition for use or method for non-therapeutic improvement of the postnatal growth trajectory or body development according to any one of the preceding claims, wherein the nutritional composition is a powder, suitable to reconstitute with water to a ready to drink formula.

## Patentansprüche

1. Verfahren zur nichttherapeutischen Verbesserung des/der postnatalen Wachstumsverlaufs oder Körperentwicklung bei einem Säugling, der von einer Mutter geboren wurde, die zum Zeitpunkt der Konzeption des Säuglings übergewichtig oder fettleibig ist, durch Verabreichung einer Nährstoffzusammensetzung, die aus einer Säuglingsanfangsnahrung und einer Folgenahrung ausgewählt ist, die Kohlenhydrate, Protein und Lipid umfasst, wobei
i) das Lipid Linolsäure und Alpha-Linolensäure in einem Gewichtsverhältnis von 2 bis 20 umfasst,
ii) das Lipid mindestens 10 Gew.-% Palmitinsäure umfasst, bezogen auf das Gesamtlipid, und mindestens 15 Gew.-% dieser Palmitinsäure an der sn-2-Position eines Triglycerids verestert sind, bezogen auf die Gesamtpalmitinsäure, und wobei die Nährstoffzusammensetzung Lipidglobuli umfasst, die Folgendes aufweisen:
a) einen Modendurchmesser, bezogen auf das Volumen, von mindestens 1,0 µm und/oder einen Durchmesser von 2 bis 12 µm in einer Menge von mindestens 45 Volumen-%, bezogen auf das Gesamtlipid, und
b) wobei die Lipidglobuli zumindest teilweise auf der Oberfläche mit Phospholipiden überzogen sind, wobei die Menge an Phospholipiden, die in der Nährstoffzusammensetzung vorhanden sind, von 0,5 bis 20 Gew.-% Phospholipide beträgt, bezogen auf die Gesamtlipide.

2. Nährstoffzusammensetzung, die aus einer Säuglingsanfangsnahrung und einer Folgenahrung ausgewählt ist, die Kohlenhydrate, Protein und Lipid umfasst, wobei
i) das Lipid Linolsäure und Alpha-Linolensäure in einem Gewichtsverhältnis von 2 bis 20 umfasst,
ii) das Lipid mindestens 10 Gew.-% Palmitinsäure umfasst, bezogen auf das Gesamtlipid, und mindestens 15 Gew.-% dieser Palmitinsäure an der sn-2-Position eines Triglycerids verestert sind, bezogen auf die Gesamtpalmitinsäure, und wobei die Nährstoffzusammensetzung Lipidglobuli umfasst, die Folgendes aufweisen:
a) einen Modendurchmesser, bezogen auf das Volumen, von mindestens 1,0 µm und/oder einen Durchmesser von 2 bis 12 µm in einer Menge von mindestens 45 Volumen-%, bezogen auf das Gesamtlipid und
b) wobei die Lipidglobuli zumindest teilweise auf der Oberfläche mit Phospholipiden überzogen sind, wobei die Menge an Phospholipiden, die in der Nährstoffzusammensetzung vorhanden sind, von 0,5 bis 20 Gew.-% Phospholipide beträgt, bezogen auf die Gesamtlipide,
zur Verwendung bei der Vorbeugung oder Verringerung des Risikos eines/einer unausgeglichenen postnatalen Wachstumsverlaufs oder Körperentwicklung bei einem Säugling, bei dem das Risiko eines/einer unausgeglichenen postnatalen Wachstumsverlaufs oder Körperentwicklung besteht, wobei der besagte Säugling von einer Mutter geboren wurde, die zum Zeitpunkt der Konzeption des Säuglings übergewichtig oder fettleibig ist.

3. Verfahren zur nichttherapeutischen Verbesserung des/der postnatalen Wachstumsverlaufs oder Körperentwicklung nach Anspruch 1, wobei die Verbesserung mit dem/der postnatalen Wachstumsverlauf oder Körperentwicklung bei einem Säugling verglichen wird, der von einer Mutter geboren wurde, die zum Zeitpunkt der Konzeption des Säuglings übergewichtig oder fettleibig ist, der mit einer Säuglingsanfangsnahrung oder einer Folgenahrung gefüttert wird, die Kohlenhydrate, Protein und Lipid umfasst und mindestens 10 Gew.-% Palmitinsäure umfasst, bezogen auf das Gesamtlipid, wobei sich jedoch weniger als 15 % der Palmitinsäurereste in der sn-2-Position von Triglyceriden befinden und keine Phospholipide umfassen und Lipidglobuli mit einem Modendurchmesser, bezogen auf das Volumen, von etwa 0,5 µm aufweisen.

4. Nährstoffzusammensetzung zur Verwendung nach Anspruch 2, wobei die Vorbeugung oder Verringerung des Risikos mit dem/der postnatalen Wachstumsverlauf oder Körperentwicklung bei einem Säugling verglichen wird, der von einer Mutter geboren wurde, die zum Zeitpunkt der Konzeption des Säuglings übergewichtig oder fettleibig ist, der mit einer Säuglingsanfangsnahrung oder einer Folgenahrung gefüttert wird, die Kohlenhydrate, Protein und Lipid umfasst und mindestens 10 Gew.-% Palmitinsäure umfasst, bezogen auf das Gesamtlipid, wobei sich jedoch weniger als 15 % der Palmitinsäurereste in der sn-2-Position von Triglyceriden befinden und keine Phospholipide umfassen und Lipidglobuli mit einem Modendurchmesser, bezogen auf das Volumen, von etwa 0,5 µm aufweisen.

5. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Nährstoffzusammensetzung weniger als 15 Gew.-% Linolsäure und mehr als 1 Gew.% Alpha-Linolensäure umfasst, bezogen auf das Gesamtlipid.

6. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei der Wachstumsverlauf oder die Körperentwicklung der Verlauf oder die Entwicklung des Körpermasseindex (BMI) ist.

7. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Verwendung zum Verringern des BMI-Anstiegs des Säuglings dient.

8. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei der Wachstumsverlauf oder die Körperentwicklung der Verlauf oder die Entwicklung des Körpermasseindex (BMI) in den ersten 12 Lebensmonaten des Säuglings ist.

9. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei der Säugling ein termingeborener Säugling ist.

10. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Phospholipide mindestens 15 Gew.-% Sphingomyelin umfassen, bezogen auf die Gesamtphospholipide.

11. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Nährstoffzusammensetzung Phospholipide umfasst, die aus der Milchfettkügelchenmembran (MFGM) stammen oder einen Teil davon bilden oder als MFGM bereitgestellt werden, vorzugsweise Kuhmilch-MFGM.

12. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Nährstoffzusammensetzung 0,5 bis 20 Gew.-% Phospholipide umfasst, die aus Säugetiermilch stammen, bezogen auf das Gesamtlipid.

13. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei mindestens 30 Gew.-% der Palmitinsäure an der sn-2-Position eines Triglycerids verestert sind, bezogen auf die Gesamtpalmitinsäure.

14. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Lipidglobuli mindestens 40 Gew.-% Triglyceride pflanzlichen Ursprungs umfassen.

15. Nährstoffzusammensetzung zur Verwendung oder Verfahren zur nichttherapeutischen Verbesserung des/der postnatalen Wachstumsverlaufs oder Körperentwicklung nach einem der vorhergehenden Ansprüche, wobei die Nährstoffzusammensetzung ein Pulver ist, das geeignet ist, um mit Wasser zu einer trinkfertigen Formel rekonstituiert zu werden.

## Revendications

1. Procédé d'amélioration non thérapeutique de la trajectoire de croissance postnatale ou du développement corporel d'un nourrisson né d'une mère qui est en surpoids ou obèse au moment de la conception du nourrisson par l'administration d'une composition nutritionnelle sélectionnée à partir d'une formule pour nourrissons et une formule de suite comprenant des carbohydrates, des protéines et des lipides, où
i) les lipides comprennent de l'acide linoléique et de l'acide alpha-linolénique dans un rapport pondéral de 2 à 20,
ii) les lipides comprennent au moins 10 % en poids d'acide palmitique sur la base des lipides totaux, et au moins 15 % en poids de cet acide palmitique est estérifié à la position sn-2 d'un triglycéride sur la base de l'acide palmitique total, et où la composition nutritionnelle comprend des globules lipidiques qui ont
a) un diamètre de mode, sur la base d'un volume d'au moins 1,0 µm et/ou un diamètre de 2 à 12 µm dans une quantité d'au moins 45 % en volume sur la base des lipides totaux et
b) les globules lipidiques sont au moins partiellement recouverts en surface de phospholipides, la quantité de phospholipides présents dans la composition nutritionnelle étant de 0,5 à 20 % en poids de phospholipides sur la base des lipides totaux.

2. Composition nutritionnelle sélectionnée à partir d'une formule pour nourrissons et une formule de suite comprenant des carbohydrates, des protéines et des lipides, où
i) les lipides comprennent de l'acide linoléique et de l'acide alpha-linolénique dans un rapport pondéral de 2 à 20,
ii) les lipides comprennent au moins 10 % en poids d'acide palmitique sur la base des lipides totaux, et au moins 15 % en poids de cet acide palmitique est estérifié à la position sn-2 d'un triglycéride sur la base de l'acide palmitique total, et où la composition nutritionnelle comprend des globules lipidiques qui ont
a) un diamètre de mode, sur la base d'un volume d'au moins 1,0 µm et/ou un diamètre de 2 à 12 µm dans une quantité d'au moins 45 % en volume sur la base des lipides totaux et
b) les globules lipidiques sont au moins partiellement recouverts en surface de phospholipides, la quantité de phospholipides présents dans la composition nutritionnelle étant de 0,5 à 20 % en poids de phospholipides sur la base des lipides totaux,
pour l'utilisation dans la prévention ou la réduction du risque d'une trajectoire de croissance postnatale déséquilibrée ou d'un développement corporel déséquilibré chez un nourrisson à risque d'avoir une trajectoire de croissance postnatale déséquilibrée ou un développement corporel déséquilibré, ledit nourrisson étant né d'une mère qui est en surpoids ou obèse au moment de la conception du nourrisson.

3. Procédé d'amélioration non thérapeutique de la trajectoire de croissance postnatale ou du développement corporel selon la revendication 1, où l'amélioration est comparée à la trajectoire de croissance postnatale ou au développement corporel chez un nourrisson né d'une mère qui est en surpoids ou obèse au moment de la conception du nourrisson nourri avec une formule pour nourrissons ou une formule de suite comprenant des carbohydrates, des protéines et des lipides, et comprenant au moins 10 % en poids d'acide palmitique sur la base des lipides totaux, cependant avec moins de 15 % des résidus d'acide palmitique étant dans la position sn-2 des triglycérides et ne comprenant pas de phospholipides et ayant des globules lipidiques avec un diamètre de mode, sur la base du volume, d'environ 0,5 µm.

4. Composition nutritionnelle pour l'utilisation selon la revendication 2, où la prévention ou la réduction du risque est comparée à la trajectoire de croissance postnatale ou au développement corporel d'un nourrisson né d'une mère qui est en surpoids ou obèse au moment de la conception du nourrisson nourri d'une formule pour nourrissons ou d'une formule de suite comprenant des carbohydrates, des protéines et des lipides, et comprenant au moins 10 % en poids d'acide palmitique sur la base des lipides totaux, cependant avec moins de 15 % des résidus d'acide palmitique étant dans la position sn-2 des triglycérides et ne comprenant pas de phospholipides et ayant des globules lipidiques avec un diamètre de mode, sur la base du volume, d'environ 0,5 µm.

5. Composition nutritionnelle pour l'utilisation ou procédé pour l'amélioration non thérapeutique de la trajectoire de croissance postnatale ou du développement corporel selon l'une quelconque des revendications précédentes, où la composition nutritionnelle comprend moins de 15 % en poids d'acide linoléique et plus de 1 % en poids d'acide alpha-linolénique sur la base des lipides totaux.

6. Composition nutritionnelle pour l'utilisation ou procédé pour l'amélioration non thérapeutique de la trajectoire de croissance postnatale ou du développement corporel selon l'une quelconque des revendications précédentes, où la trajectoire de croissance ou le développement corporel est la trajectoire ou le développement de l'indice de masse corporelle (IMC).

7. Composition nutritionnelle pour l'utilisation ou procédé pour l'amélioration non thérapeutique de la trajectoire de croissance postnatale ou du développement corporel selon l'une quelconque des revendications précédentes, où l'utilisation est pour réduire l'augmentation de l'IMC du nourrisson.

8. Composition nutritionnelle pour l'utilisation ou procédé pour l'amélioration non thérapeutique de la trajectoire de croissance postnatale ou du développement corporel selon l'une quelconque des revendications précédentes, où la trajectoire de croissance ou le développement corporel est la trajectoire ou le développement de l'indice de masse corporelle (IMC) dans les 12 premiers mois de la vie du nourrisson.

9. Composition nutritionnelle pour l'utilisation ou procédé pour l'amélioration non thérapeutique de la trajectoire de croissance postnatale ou du développement corporel selon l'une quelconque des revendications précédentes, où le nourrisson est un nourrisson né à terme.

10. Composition nutritionnelle pour l'utilisation ou procédé pour l'amélioration non thérapeutique de la trajectoire de croissance postnatale ou du développement corporel selon l'une quelconque des revendications précédentes, où les phospholipides comprennent au moins 15 % en poids de sphingomyéline sur la base des phospholipides totaux.

11. Composition nutritionnelle pour l'utilisation ou procédé pour l'amélioration non thérapeutique de la trajectoire de croissance postnatale ou du développement corporel selon l'une quelconque des revendications précédentes, où la composition nutritionnelle comprend des phospholipides qui sont dérivés de ou font partie de la membrane des globules gras du lait ( MFGM), ou sont fournis sous forme de MFGM, de préférence des MFGM de lait de vache.

12. Composition nutritionnelle pour l'utilisation ou procédé pour l'amélioration non thérapeutique de la trajectoire de croissance postnatale ou du développement corporel selon l'une quelconque des revendications précédentes, où la composition nutritionnelle comprend 0,5 à 20 % en poids de phospholipides dérivés du lait de mammifère sur la base des lipides totaux.

13. Composition nutritionnelle pour l'utilisation ou procédé pour l'amélioration non thérapeutique de la trajectoire de croissance postnatale ou du développement corporel selon l'une quelconque des revendications précédentes, où au moins 30 % en poids d'acide palmitique est estérifié à la position sn-2 d'un triglycéride sur la base de l'acide palmitique total.

14. Composition nutritionnelle pour l'utilisation ou procédé pour l'amélioration non thérapeutique de la trajectoire de croissance postnatale ou du développement corporel selon l'une quelconque des revendications précédentes, où les globules lipidiques comprennent au moins 40 % en poids de triglycérides d'origine végétale.

15. Composition nutritionnelle pour l'utilisation ou procédé pour l'amélioration non thérapeutique de la trajectoire de croissance postnatale ou du développement corporel selon l'une quelconque des revendications précédentes, où la composition nutritionnelle est une poudre, appropriée pour être reconstituée avec de l'eau en une formule prête à boire.
